# EUROPÄISCHE PATENTANMELDUNG

(11) **EP 1 400 828 A1**
(43) Veröffentlichungstag der Anmeldung: **24.03.2004**
(21) Anmeldenummer: 02020833.6
(22) Anmeldetag: 17.09.2002
(51) Int. Cl.: G02B 21/00, G02B 7/08, G02B 21/24, A61B 19/00

(54) **Operationsmikroskopsystem**

(71) Anmelder: Möller-Wedel GmbH, 22880 Wedel (DE)
(72) Erfinder: Schmidt, Martin, Dr., 23611 Bad Schwartau (DE); Schalt, Peter, 25436 Moorrege (DE)
(74) Vertreter: Glawe, Delfs, Moll & Partner

(57) **Zusammenfassung**

Das Operationsmikroskopsystem mit einem Operationsmikroskop (11), einem Trägersystem (14) für das Mikroskop (11) und mindestens einem durch einen Schalter (16) betätigbaren Antriebselement (31) für Bewegungen und/oder Fokussierung des Mikroskops (11), zeichnet sich dadurch aus, daß der Schalter (16) als sterilisierbarer Handschalter ausgebildet ist, der am oder auf dem Operationstisch (17) anzuordnen ist.

## Beschreibung

Die Erfindung betrifft ein Operationsmikroskopsystem mit einem Operationsmikroskop, einem Trägersystem für das Mikroskop und mindestens einem durch einen Schalter betätigbaren Antriebselement für Bewegungen und/oder Fokussierung des Mikroskops.

Für Operationen an feinen biologischen Strukturen werden Operationsmikroskope benötigt. Insbesondere für neurochirurgische Eingriffe ist es erforderlich, daß die Operationsmikroskope im Raum bewegt werden können, ohne daß der Chirurg dabei unsteril wird.

Es existieren Operationsmikroskope, die durch bewegliche Trägersysteme und eine bewegliche Mikroskopaufhängung eine Bewegung um 3 Translationsachsen und 3 Drehachsen zulassen. Dazu haben diese Mikroskope Handgriffe, die wie auch der Mikroskopkörner selbst durch sterile Folien abgedeckt werden. Der Chirurg ergreift den Handgriff und drückt auf einen Auslöseknopf, wodurch sich Bremsen in sämtlichen Achsen lösen und das Mikroskop frei im Raum positionierbar und drehbar wird. Nachteilig ist dabei, daß der Chirurg meist das Operationsinstrument aus der Hand geben muß und daß die Positionierung wegen der Freiheit aller Achsen schwierig ist.

In einer anderen Ausführung (DE 43 11 467) besitzen Operationsmikroskope Motoren, die einige der Bewegungen über Fernsteuerung auslösen lassen. So werden in der Ophthalmologie die horizontale XY-Bewegung und zum Fokussieren der vertikale Z-Bewegung meist durch einen Fußschalter ausgelöst. Auch für die Neurochirurgie gibt es Operationsmikroskope, die die Rotationen des Mikroskops durch Fußschalter oder Schalter am Handgriff des Mikroskops ansteuern lassen. Bei Fußschaltern braucht für Korrekturen das Operationsinstrument nicht aus der Hand gegeben zu werden, jedoch können Fußschalter nur im Sitzen verwendet werden. Befindet sich der Schalter am Handgriff, muß zur Bedienung das Operationsinstrument aus der Hand gegeben werden.

Es gibt auch voll robotisch gesteuerte Operationsmikroskope, bei denen alle Translations- und Rotationsbewegungen elektrisch gesteuert werden können. Auch hier erfolgt die Steuerung meist über Handschalter, wodurch sich die oben erwähnten Nachteile nicht vermeiden lassen.

Es gab Versuche, Operationsmikroskope über Sprachsignale, Kopfbewegungen oder Augenbewegungen zu steuern. Diese Techniken haben sich jedoch nicht durchgesetzt, da zu viel Konzentration für den Chirurgen erforderlich ist und die Gefahr der Misinterpretation gegebener Signale besteht.

Aufgabe der Erfindung ist die Schaffung eines Operationsmikroskops der eingangs genannten Art, bei dem der Schalter für Bewegungen und/oder Fokussierung des Mikroskops auf besonders vorteilhafte Weise bedient werden kann.

Die erfindungsgemäße Lösung besteht darin, daß der Schalter als sterilisierbarer Handschalter ausgebildet ist, der am oder auf dem Operationstisch anzuordnen ist.

Da der Schalter am oder auf dem Operationstisch anzuordnen ist, ist er in großer Nähe zum Patienten bzw. der Operationsstelle angeordnet, so daß der Operateur den Schalter betätigen kann, ohne daß Operationsinstrument aus der Hand zu nehmen. Seine Hand wird dabei auch nicht verunreinigt, da der Schalter sterilisierbar ist. Man hat also den Vorteil der besonders genauen Steuerung von Hand ohne die Nachteile der bisherigen Steuerungen. Erstaunlicherweise hat es,sich erwiesen, daß solche Schalter einerseits auf sehr geringe Kräfte reagieren können, so daß sie z. B. mit dem kleinen Finger betätigt werden können, andererseits aber doch so robust sind, daß sie sterilisiert werden können. Durch diesen Handschalter ist also eine problemlose Steuerung der Bewegungen und/oder der Fokussierung des Mikroskops möglich. Dieses braucht vor der Operation nur grob eingestellt werden, was entweder von Hand oder aber maschinell über Schalter geschehen kann, die nicht sterilisierbar sein müssen. Die eigentliche Feineinstellung wird dann aber während der Operation über den Handschalter vorgenommen bzw. nachgestellt.

Der Schalter könnte mit einem entsprechend schweren Sockel ausgebildet sein, so daß er nicht leicht auf den Operationstisch verschoben werden kann. Besonders zweckmäßig ist es aber, wenn der Schalter Einrichtungen zum Befestigen am Operationstisch aufweist, da dann die Gefahr nicht besteht, daß der Schalter versehentlich verschoben wird.

Wenn ein entsprechend sterilisierbares Kabel und, falls erforderlich, auch ein entsprechend sterilisierbarer Stecker vorgesehen ist, kann vorgesehen sein, daß der Schalter mit einem Kabel mit dem Antriebselement bzw. dessen Versorgungseinheit verbunden ist.

Die Sterilisationsprobleme bei dem Kabel bzw. dem Stecker werden vermieden, wenn der Schalter drahtlos mit dem An triebselement bzw. dessen Versorgungseinheit verbunden ist. Als drahtlose Verbindung kommen dabei insbesondere eine Infrarot-, Ultraschall- oder Funkverbindung in Frage.

Zweckmäßigerweise sind auch die Einrichtungen zum Befestigen am Operationstisch sterilisierbar und weisen eine Klemmzwinge sowie ein verstellbares Verbindungselement zwischen Klemmzwinge und Schalter auf.

Das Verbindungselement kann eine Kugelkette, einen Schwanenhals oder eine Anordnung von durch Gelenken oder Klemmen verbundenen Stangen oder Rohren aufweisen.

Besonders zweckmäßig ist es, wenn der Schalter einen Sockel und einen sich vom Sockel erstreckendes hakenförmiges oder hinterschnittenes Betätigungselement aufweist. Ein solches Betätigungselement kann heruntergedrückt oder durch Hintergreifen angehoben werden. Dadurch könnte die Fokussierung in den beiden Richtungen gesteuert werden. Hierfür ist es besonders zweckmäßig, wenn das Betätigungselement pilzförmig ist. Diese Pilzform ist auch besonders zweckmäßig, wenn das Betätigungselement in zwei zueinander senkrecht im wesentlichen horizontalen Richtungen und in im wesentlichen vertikale Richtung bewegbar ist und sechs entsprechende Schaltfunktionen hat. Das pilzförmige Betätigungselement kann also horizontal in zwei zueinander senkrechten Richtungen vor- oder zurückbewegt werden und dadurch die Position bzw. Schwenkung des Operationsmikroskops in zwei zueinander senkrechten horizontalen Ebenen X, Y steuern. Durch Anheben oder Niederdrükken des Betätigungselements kann dann die Fokussierung erfolgen. Selbstverständlich könnte das Betätigungselement aber auch andere Form haben, z. B. T-förmig sein. In diesem Falle wäre lediglich die in X- oder Y-Richtung auszuübende Betätigungskraft nicht mehr gleich.

Zweckmäßigerweise sind dabei die Betätigungsrichtungen in X- und Y-Richtungen parallel zu den Schwenkrichtungen des Mikroskops. Sollte dies bei der Montage des Schalters nicht zu erreichen sein, ist es zweckmäßig, wenn der Schalter um eine vartikale Achse einstellbar ist, um diese Parallelität zu erreichen.

Die Erfindung wird im folgenden anhand einer vorteilhaften Ausführungsform unter Bezugnahme auf die beigefügten Zeichnungen beschrieben. Es zeigen:
- Fig. 1: in schematischer und perspektivischer Ansicht das Prinzip des Operationsmikroskopsystems;
- Fig. 2: eine Ausführungsform des Handschalters; und
- Fig. 3: eine andere Ausführungsform des Handschalters.

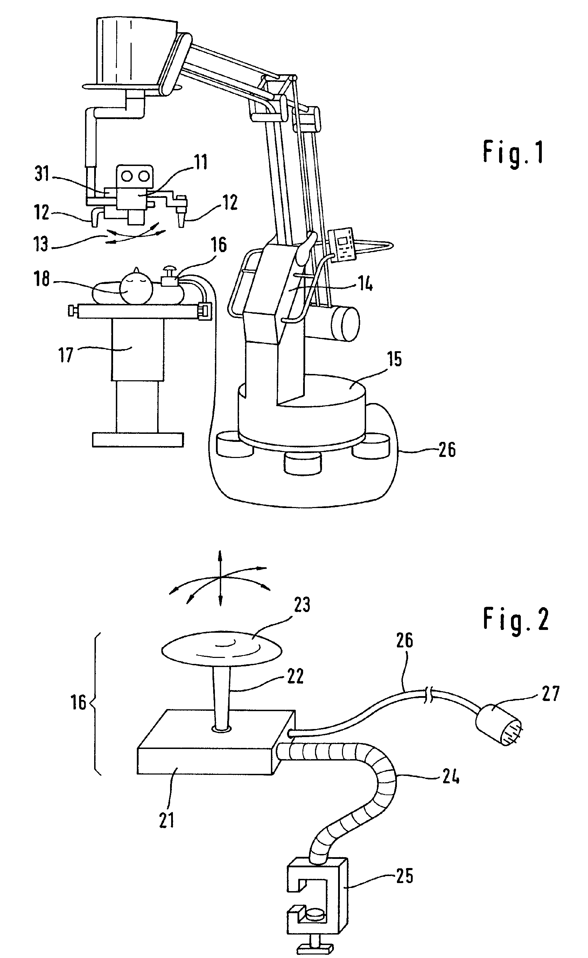

In Fig. 1 stellt das Gerät 11 das Operationsmikroskop dar, das üblicherweise mit Handgriffen 12 ausgestattet ist. Dabei ist das Operationsmikroskop mit Motoren für die Rotationsbewegungen 13 und die Fokussierung ausgestattet, die bei 31 angedeutet sind. Zur Aufhängung des Operationsmikroskops 11 im Raum dient das Trägersystem 14, das eine elektrische Versorgungseinheit 15 enthält mit einem elektrischen Anschluß für die Motoren. Ein Handschalter 16 ist am Operationstisch 17 befestigt, auf dem sich der Patient 18 befindet. Durch die Befestigung am Operationstisch 17 kann der Handschalter 16 nahe am Operationsfeld angebracht und dadurch vom Chirurgen während der Operation bedient werden, ohne das Operationsinstrument aus der Hand zu legen. Der Handschalter ist über ein Verbindungskabel 26 oder eine drahtlose Verbindung 32 mit der Versorgungseinheit 15 für die Motoren verbunden.

In Fig. 2 ist eine Ausführungsform des Handschalters 16 dargestellt. Auf einem Sockel 21 ist ein Hebel 22 angebracht, der am oberen Ende eine Kalotte 23, eine Platte oder einen Haken enthält. Dabei ist der Hebel 22 bzw. die Kalotte 23, die Platte oder der Haken so geformt, daß der Chirurg mit dem kleinen Finger sowohl den Hebel lateral bewegen als auch senkrecht drücken oder heben kann. Durch die laterale Bewegung wird vorzugsweise die Rotationsbewegung 13 gesteuert, die bei Blick durch das Mikroskop als XY-Bewegung erscheint. Durch Drücken und Heben des Hebels 22 wird der Fokus betätigt.

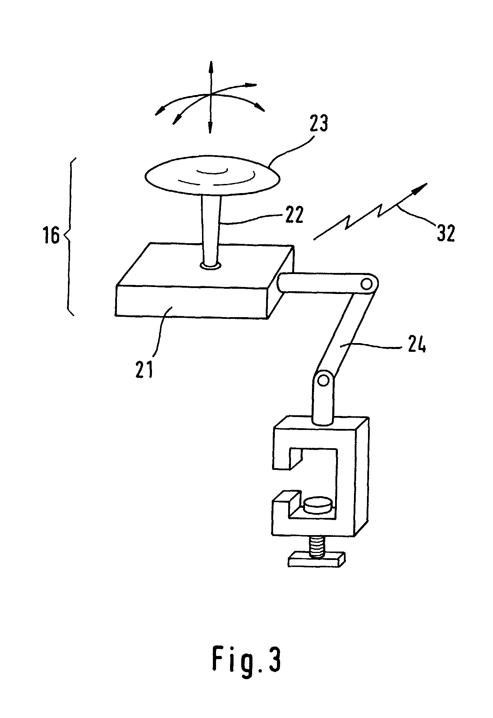

Der Handschalter 16 ist über einen Arm 24 mit einer Klemmspange 25 verbunden. Der Arm 24 enthält mehrere Gelenke, damit der Handschalter 16 optimal zum Operationsfeld positioniert werden kann. Die Gelenke sind dabei feststellbar. Beispielsweise kann der Arm 24 aus einer Kugelkette bestehen, die über ein innen liegendes spannbares Drahtseil in einer bestimmten Form gehalten wird. Auch kann der Arm 24 aus einem "Schwanenhals" bestehen, der steif genug ist, um während der Operation seine Form zu behalten. Möglich ist es aber auch, den Arm 24 aus mehreren Stangen oder Rohren auszuführen, die durch Gelenke oder Klemmen miteinander verbunden sind, wie dies in Fig. 3 angedeutet ist. Die Klemmspange 25 ist so ausgeführt, daß sie an den üblichen Klemmschienen an Operationstischen oder an anderen am Operationstisch angebrachten Teilen, z. B. dem Kopfhalter angebracht werden kann.

Von dem Handschalter geht ein Kabel 26 aus, das über einen Stecker 27 mit der Versorgungseinheit des Stativs verbunden werden kann. So können Schaltsignale die Rotationen 13 des Mikroskops 11 sowie dessen Fokussierung bewirken. Es ist auch möglich, zwischen dem Stecker 27 und der Versorgungseinheit 15 ein Zwischenkabel anzubringen. In einer anderen Ausführungsform kann man auf das Kabel 26 auch ganz verzichten, wenn der Handschalter 16 eine Spendeeinheit für elektromagnetische Wellen, Lichtpulse oder Ultraschallsignale enthält und an der Versorgungseinheit 15 ein Empfänger für diese Signale angebracht ist. Dieser Fall ist in Fig. 3 bei 32 angedeutet.

Es ist wichtig, daß der Handschalter 16 sowie der Arm 24 sterilisierbar ausgeführt sind. Anderenfalls müßte während der Operation der Handschalter 16 durch sterilisierbare Folien abgedeckt werden, wodurch das direkte Gefühl verloren ginge. Vorzugsweise enthält der Handschalter 16 Hochtemperatur feste Schaltelemente oder besteht gänzlich aus keramischen Materialien und Metallen. Wenn außerdem der Arm 24, die Klemmspange 25, das Kabel 26 und der Stecker 27 ebenfalls für hohe Temperaturen ausgelegt sind, z. B. durch Silikon- und Teflonisolierung der elektrischen Teile, ist eine Sterilisation durch Heißluft (165°C) oder Autoklavieren (134°) durchführbar. Anderenfalls muß der Handschalter 16 auf chemischem Wege (Flüssigkeitsimmersion, Gassterilisation) oder durch Plasmasterilisation sterilisiert werden.

## Patentansprüche

1. Operationsmikroskopsystem mit einem Operationsmikroskop (11), einem Trägersystem (14) für das Mikroskop (11) und mindestens einem durch einen Schalter (16) betätigbaren Antriebselement (31) für Bewegungen und/oder Fokussierung des Mikroskops (11), **dadurch gekennzeichnet, daß** der Schalter (16) als sterilisierbarer Handschalter ausgebildet ist, der am oder auf dem Operationstisch (17) anzuordnen ist.

2. Operationsmikroskopsystem nach Anspruch 1, **dadurch gekennzeichnet, daß** der Schalter (16) Einrichtungen (24, 25) zum Befestigen am Operationstisch (17) oder an Teilen, die am Operationstisch angebracht sind, aufweist.

3. Operationsmikroskopsystem nach Anspruch 1 oder 2, **dadurch gekennzeichnet, daß** der Schalter (16) mit einem Kabel (26) mit dem Antriebselement (31) bzw. dessen Versorgungseinheit (15) verbunden ist.

4. Operationsmikroskopsystem nach Anspruch 1 oder 2, **dadurch gekennzeichnet, daß** der Schalter (16) drahtlos (32) mit dem Antriebselement (31) bzw. dessen Versorgungseinheit (15) verbunden ist.

5. Operationsmikroskopsystem nach einem der Ansprüche 2 bis 4, **dadurch gekennzeichnet, daß** die Einrichtungen (24, 25) zum Befestigen am Operationstisch (17) oder an Teilen, die am Operationstisch angebracht sind, sterilisierbar sind und eine Klemmzwinge (25) sowie ein verstellbares Verbindungselement (24) zwischen Klemmzwinge (25) und Schalter (16) aufweist.

6. Operationsmikroskopsystem nach Anspruch 5, **dadurch gekennzeichnet, daß** das Verbindungselement (24) eine Kugelkette, einen Schwanenhals oder eine Anordnung von durch Gelenke oder Klemmen verbundenen Stangen oder Rohren aufweist.

7. Operationsmikroskopsystem nach einem der Ansprüche 1 bis 6, **dadurch gekennzeichnet, daß** der Schalter (16) einen Sockel (21) und ein sich vom Sockel erstreckendes hakenförmiges oder hinterschnittenes Betätigungselement (23) aufweist.

8. Operationsmikroskopsystem nach Anspruch 7, **dadurch gekennzeichnet, daß** das Betätigungselement (23) pilzförmig ist.

9. Operationsmikroskopsystem nach einem der Ansprüche 7 oder 8, **dadurch gekennzeichnet, daß** das Betätigungselement (23) in zwei zueinander senkrechten im wesentlichen horizontalen Richtungen und in im wesentlichen vertikaler Richtung bewegbar ist und sechs entsprechende Schaltfunktionen hat.

10. Operationsmikroskopsystem nach einem der Ansprüche 1 bis 9, **dadurch gekennzeichnet, daß** der Schalter (16) um eine vertikale Achse einstellbar ist.
